# EUROPEAN PATENT APPLICATION

(11) **EP 3 081 639 A1**
(43) Date of publication of application: **19.10.2016**
(21) Application number: 15163499.5
(22) Date of filing: 14.04.2015
(51) Int. Cl.: C12N 9/00, C12N 15/09, C12P 13/04, C12P 13/16

(54) **Acyl amino acid production**

(71) Applicant: Evonik Degussa GmbH, 45128 Essen (DE)
(72) Inventor: SCHAFFER, Steffen, 45699 Herten (DE); GRAMMANN, Katrin, 45739 Oer-Erkenschwick (DE); REINECKE, Liv, 45134 Essen (DE)

(57) **Abstract**

The present invention relates to a microbial cell for producing at least one acyl amino acid, wherein the cell is genetically modified to comprise;
- a first genetic mutation that enables the cell to produce at least one acyl amino acid and;
- a second genetic mutation that enables the cell to decrease glutamate breakdown relative to the wild type cell.

SEQUENCE LISTING

<110>  Evonik Industries AG

<120>  ACYL AMINO ACID PRODUCTION

<130>  2014P00319EP

<160>  8

<170>  PatentIn version 3.5

<210>  1
<211>  561
<212>  PRT
<213>  Artificial Sequence

<220>
<223>  FadD Acyl-CoA synthase AA

<400>  1

Met Lys Lys Val Trp Leu Asn Arg Tyr Pro Ala Asp Val Pro Thr Glu
1               5                   10                  15

Ile Asn Pro Asp Arg Tyr Gln Ser Leu Val Asp Met Phe Glu Gln Ser
            20                  25                  30

Val Ala Arg Tyr Ala Asp Gln Pro Ala Phe Val Asn Met Gly Glu Val
        35                  40                  45

Met Thr Phe Arg Lys Leu Glu Glu Arg Ser Arg Ala Phe Ala Ala Tyr
    50                  55                  60

Leu Gln Gln Gly Leu Gly Leu Lys Lys Gly Asp Arg Val Ala Leu Met
65                  70                  75                  80

Met Pro Asn Leu Leu Gln Tyr Pro Val Ala Leu Phe Gly Ile Leu Arg
                85                  90                  95

Ala Gly Met Ile Val Val Asn Val Asn Pro Leu Tyr Thr Pro Arg Glu
            100                 105                 110

Leu Glu His Gln Leu Asn Asp Ser Gly Ala Ser Ala Ile Val Ile Val
        115                 120                 125

Ser Asn Phe Ala His Thr Leu Glu Lys Val Val Asp Lys Thr Ala Val
    130                 135                 140

Gln His Val Ile Leu Thr Arg Met Gly Asp Gln Leu Ser Thr Ala Lys
145                 150                 155                 160

Gly Thr Val Val Asn Phe Val Val Lys Tyr Ile Lys Arg Leu Val Pro

                165                 170                 175

Lys Tyr His Leu Pro Asp Ala Ile Ser Phe Arg Ser Ala Leu His Asn
            180                 185                 190

Gly Tyr Arg Met Gln Tyr Val Lys Pro Glu Leu Val Pro Glu Asp Leu
        195                 200                 205

Ala Phe Leu Gln Tyr Thr Gly Gly Thr Thr Gly Val Ala Lys Gly Ala
    210                 215                 220

Met Leu Thr His Arg Asn Met Leu Ala Asn Leu Glu Gln Val Asn Ala
225                 230                 235                 240

Thr Tyr Gly Pro Leu Leu His Pro Gly Lys Glu Leu Val Val Thr Ala
                245                 250                 255

Leu Pro Leu Tyr His Ile Phe Ala Leu Thr Ile Asn Cys Leu Leu Phe
            260                 265                 270

Ile Glu Leu Gly Gly Gln Asn Leu Leu Ile Thr Asn Pro Arg Asp Ile
        275                 280                 285

Pro Gly Leu Val Lys Glu Leu Ala Lys Tyr Pro Phe Thr Ala Ile Thr
    290                 295                 300

Gly Val Asn Thr Leu Phe Asn Ala Leu Leu Asn Asn Lys Glu Phe Gln
305                 310                 315                 320

Gln Leu Asp Phe Ser Ser Leu His Leu Ser Ala Gly Gly Gly Met Pro
                325                 330                 335

Val Gln Gln Val Val Ala Glu Arg Trp Val Lys Leu Thr Gly Gln Tyr
            340                 345                 350

Leu Leu Glu Gly Tyr Gly Leu Thr Glu Cys Ala Pro Leu Val Ser Val
        355                 360                 365

Asn Pro Tyr Asp Ile Asp Tyr His Ser Gly Ser Ile Gly Leu Pro Val
    370                 375                 380

Pro Ser Thr Glu Ala Lys Leu Val Asp Asp Asp Asp Asn Glu Val Pro
385                 390                 395                 400

Pro Gly Gln Pro Gly Glu Leu Cys Val Lys Gly Pro Gln Val Met Leu
                405                 410                 415

Gly Tyr Trp Gln Arg Pro Asp Ala Thr Asp Glu Ile Ile Lys Asn Gly
            420                 425                 430

Trp Leu His Thr Gly Asp Ile Ala Val Met Asp Glu Glu Gly Phe Leu
        435                 440                 445

Arg Ile Val Asp Arg Lys Lys Asp Met Ile Leu Val Ser Gly Phe Asn
    450                 455                 460

Val Tyr Pro Asn Glu Ile Glu Asp Val Val Met Gln His Pro Gly Val
465                 470                 475                 480

Gln Glu Val Ala Ala Val Gly Val Pro Ser Gly Ser Ser Gly Glu Ala
                485                 490                 495

Val Lys Ile Phe Val Val Lys Lys Asp Pro Ser Leu Thr Glu Glu Ser
            500                 505                 510

Leu Val Thr Phe Cys Arg Arg Gln Leu Thr Gly Tyr Lys Val Pro Lys
        515                 520                 525

Leu Val Glu Phe Arg Asp Glu Leu Pro Lys Ser Asn Val Gly Lys Ile
    530                 535                 540

Leu Arg Arg Glu Leu Arg Asp Glu Ala Arg Gly Lys Val Asp Asn Lys
545                 550                 555                 560

Ala

<210>  2
<211>  1680
<212>  DNA
<213>  Artificial Sequence

<220>
<223>   fadD (an acyl-CoA synthetase)

<400>  2
ttgaagaagg tttggcttaa ccgttatccc gcggacgttc cgacggagat caaccctgac      60

cgttatcaat ctctggtaga tatgtttgag cagtcggtcg cgcgctacgc cgatcaacct     120

gcgtttgtga atatggggga ggtaatgacc ttccgcaagc tggaagaacg cagtcgcgcg     180

tttgccgctt atttgcaaca agggttgggg ctgaagaaag gcgatcgcgt tgcgttgatg     240

atgcctaatt tattgcaata tccggtggcg ctgtttggca ttttgcgtgc cgggatgatc     300

gtcgtaaacg ttaacccgtt gtataccccg cgtgagcttg agcatcagct taacgatagc     360

ggcgcatcgg cgattgttat cgtgtctaac tttgctcaca cactggaaaa agtggttgat     420

aaaaccgccg ttcagcacgt aattctgacc cgtatgggcg atcagctatc tacggcaaaa 480

ggcacggtag tcaatttcgt tgttaaatac atcaagcgtt tggtgccgaa ataccatctg 540

ccagatgcca tttcatttcg tagcgcactg cataacggct accggatgca gtacgtcaaa 600

cccgaactgg tgccggaaga tttagctttt ctgcaataca ccggcggcac cactggtgtg 660

gcgaaaggcg cgatgctgac tcaccgcaat atgctggcga acctggaaca ggttaacgcg 720

acctatggtc cgctgttgca tccgggcaaa gagctggtgg tgacggcgct gccgctgtat 780

cacatttttg ccctgaccat taactgcctg ctgtttatcg aactgggtgg gcagaacctg 840

cttatcacta acccgcgcga tattccaggg ttggtaaaag agttagcgaa atatccgttt 900

accgctatca cgggcgttaa caccttgttc aatgcgttgc tgaacaataa agagttccag 960

cagctggatt tctccagtct gcatctttcc gcaggcggtg ggatgccagt gcagcaagtg 1020

gtggcagagc gttgggtgaa actgaccgga cagtatctgc tggaaggcta tggccttacc 1080

gagtgtgcgc cgctggtcag cgttaaccca tatgatattg attatcatag tggtagcatc 1140

ggtttgccgg tgccgtcgac ggaagccaaa ctggtggatg atgatgataa tgaagtacca 1200

ccaggtcaac cgggtgagct ttgtgtcaaa ggaccgcagg tgatgctggg ttactggcag 1260

cgtcccgatg ctaccgatga aatcatcaaa aatggctggt tacacaccgg cgacatcgcg 1320

gtaatggatg aagaaggatt cctgcgcatt gtcgatcgta aaaaagacat gattctggtt 1380

tccggtttta acgtctatcc caacgagatt gaagatgtcg tcatgcagca tcctggcgta 1440

caggaagtcg cggctgttgg cgtaccttcc ggctccagtg gtgaagcggt gaaaatcttc 1500

gtagtgaaaa aagatccatc gcttaccgaa gagtcactgg tgactttttg ccgccgtcag 1560

ctcacgggat acaaagtacc gaagctggtg gagtttcgtg atgagttacc gaaatctaac 1620

gtcggaaaaa ttttgcgacg agaattacgt gacgaagcgc gcggcaaagt ggacaataaa 1680

<210> 3
<211> 301
<212> PRT
<213> Artificial Sequence

<220>
<223> synUcTE (an acyl-CoA thioesterase) gene (codon-optimized)

<400> 3

Met Thr Leu Glu Trp Lys Pro Lys Pro Lys Leu Pro Gln Leu Leu Asp
1               5                   10                  15

Asp His Phe Gly Leu His Gly Leu Val Phe Arg Arg Thr Phe Ala Ile
            20                  25                  30

Arg Ser Tyr Glu Val Gly Pro Asp Arg Ser Thr Ser Ile Leu Ala Val
        35                  40                  45

Met Asn His Met Gln Glu Ala Thr Leu Asn His Ala Lys Ser Val Gly
    50                  55                  60

Ile Leu Gly Asp Gly Phe Gly Thr Thr Leu Glu Met Ser Lys Arg Asp
65                  70                  75                  80

Leu Met Trp Val Val Arg Arg Thr His Val Ala Val Glu Arg Tyr Pro
                85                  90                  95

Thr Trp Gly Asp Thr Val Glu Val Glu Cys Trp Ile Gly Ala Ser Gly
            100                 105                 110

Asn Asn Gly Met Arg Arg Asp Phe Leu Val Arg Asp Cys Lys Thr Gly
        115                 120                 125

Glu Ile Leu Thr Arg Cys Thr Ser Leu Ser Val Leu Met Asn Thr Arg
    130                 135                 140

Thr Arg Arg Leu Ser Thr Ile Pro Asp Glu Val Arg Gly Glu Ile Gly
145                 150                 155                 160

Pro Ala Phe Ile Asp Asn Val Ala Val Lys Asp Asp Glu Ile Lys Lys
                165                 170                 175

Leu Gln Lys Leu Asn Asp Ser Thr Ala Asp Tyr Ile Gln Gly Gly Leu
            180                 185                 190

Thr Pro Arg Trp Asn Asp Leu Asp Val Asn Gln His Val Asn Asn Leu
        195                 200                 205

Lys Tyr Val Ala Trp Val Phe Glu Thr Val Pro Asp Ser Ile Phe Glu
    210                 215                 220

Ser His His Ile Ser Ser Phe Thr Leu Glu Tyr Arg Arg Glu Cys Thr
225                 230                 235                 240

Arg Asp Ser Val Leu Arg Ser Leu Thr Thr Val Ser Gly Gly Ser Ser
                245                 250                 255

Glu Ala Gly Leu Val Cys Asp His Leu Leu Gln Leu Glu Gly Gly Ser
            260                 265                 270

Glu Val Leu Arg Ala Arg Thr Glu Trp Arg Pro Lys Leu Thr Asp Ser
        275                 280                 285

Phe Arg Gly Ile Ser Val Ile Pro Ala Glu Pro Arg Val

290                 295                 300

<210>  4
<211>  288
<212>  PRT
<213>  Homo sapiens

<400>  4

Met Leu Val Leu Asn Cys Ser Thr Lys Leu Leu Ile Leu Glu Lys Met
1               5                   10                  15

Leu Lys Ser Cys Phe Pro Glu Ser Leu Lys Val Tyr Gly Ala Val Met
            20                  25                  30

Asn Ile Asn Arg Gly Asn Pro Phe Gln Lys Glu Val Val Leu Asp Ser
        35                  40                  45

Trp Pro Asp Phe Lys Ala Val Ile Thr Arg Arg Gln Arg Glu Ala Glu
    50                  55                  60

Thr Asp Asn Leu Asp His Tyr Thr Asn Ala Tyr Ala Val Phe Tyr Lys
65                  70                  75                  80

Asp Val Arg Ala Tyr Arg Gln Leu Leu Glu Glu Cys Asp Val Phe Asn
                85                  90                  95

Trp Asp Gln Val Phe Gln Ile Gln Gly Leu Gln Ser Glu Leu Tyr Asp
            100                 105                 110

Val Ser Lys Ala Val Ala Asn Ser Lys Gln Leu Asn Ile Lys Leu Thr
        115                 120                 125

Ser Phe Lys Ala Val His Phe Ser Pro Val Ser Ser Leu Pro Asp Thr
    130                 135                 140

Ser Phe Leu Lys Gly Pro Ser Pro Arg Leu Thr Tyr Leu Ser Val Ala
145                 150                 155                 160

Asn Ala Asp Leu Leu Asn Arg Thr Trp Ser Arg Gly Gly Asn Glu Gln
                165                 170                 175

Cys Leu Arg Tyr Ile Ala Asn Leu Ile Ser Cys Phe Pro Ser Val Cys
            180                 185                 190

Val Arg Asp Glu Lys Gly Asn Pro Val Ser Trp Ser Ile Thr Asp Gln
        195                 200                 205

Phe Ala Thr Met Cys His Gly Tyr Thr Leu Pro Glu His Arg Arg Lys

210                 215                 220

Gly Tyr Ser Arg Leu Val Ala Leu Thr Leu Ala Arg Lys Leu Gln Ser
225                 230                 235                 240

Arg Gly Phe Pro Ser Gln Gly Asn Val Leu Asp Asp Asn Thr Ala Ser
                245                 250                 255

Ile Ser Leu Leu Lys Ser Leu His Ala Glu Phe Leu Pro Cys Arg Phe
            260                 265                 270

His Arg Leu Ile Leu Thr Pro Ala Thr Phe Ser Gly Leu Pro His Leu
        275                 280                 285

<210>  5
<211>  867
<212>  DNA
<213>  Artificial Sequence

<220>
<223>  glycine N-acyltransferase

<400>  5
atgttggtgc taaactgttc taccaaatta ctgatactgg agaaaatgtt gaagagttgc      60

tttcctgaat cactcaaggt ttacggagcg gtgatgaaca taaatcgtgg gaaccccttt     120

caaaaggaag tggtgttgga ttcatggccg gatttcaaag ctgttatcac ccgacgacaa     180

agagaggctg agacagataa ccttgatcat tatactaatg cctatgctgt gttctacaag     240

gatgtcaggg cttatcgaca gctattggaa gaatgtgatg tttttaactg ggaccaagtt     300

tttcaaatac aagggctgca gagtgagtta tatgatgttt ccaaagcggt tgccaattca     360

aagcagttga atataaagct aacttccttc aaggctgttc atttttctcc tgtttcatct     420

ctgccagata ccagtttcct caaggggcct tccccacgac taacctacct gagtgttgcc     480

aatgcggatc tactcaaccg gacttggtcc cggggaggca atgaacaatg tctccggtac     540

atcgccaacc tcatctcctg cttccctagt gtgtgtgtcc gggatgagaa gggaaacccg     600

gtctcctggt ccatcacaga ccagtttgcc accatgtgcc atggctacac cctgccagaa     660

catcgcagga aaggttacag ccggctggtg gccctcacgc tggccaggaa gttgcaaagc     720

cggggattcc cctctcaggg gaacgtcctg gatgacaaca cggcgtctat aagcctcctg     780

aagagtctcc atgctgagtt cttgccttgt cgcttccaca ggcttattct cacccctgcg     840

actttctctg gcctgcctca cctctag                                         867

<210>  6
<211>  230
<212>  PRT
<213>  Pseudomonas putida

<400>  6

Met Ser Phe Ser Asn Tyr Lys Val Ile Ala Met Pro Val Leu Val Ala
1               5                   10                  15

Asn Phe Val Leu Gly Ala Ala Thr Ala Trp Ala Asn Glu Asn Tyr Pro
            20                  25                  30

Ala Lys Ser Ala Gly Tyr Asn Gln Gly Asp Trp Val Ala Ser Phe Asn
        35                  40                  45

Phe Ser Lys Val Tyr Val Gly Glu Glu Leu Gly Asp Leu Asn Val Gly
    50                  55                  60

Gly Gly Ala Leu Pro Asn Ala Asp Val Ser Ile Gly Asn Asp Thr Thr
65                  70                  75                  80

Leu Thr Phe Asp Ile Ala Tyr Phe Val Ser Ser Asn Ile Ala Val Asp
                85                  90                  95

Phe Phe Val Gly Val Pro Ala Arg Ala Lys Phe Gln Gly Glu Lys Ser
            100                 105                 110

Ile Ser Ser Leu Gly Arg Val Ser Glu Val Asp Tyr Gly Pro Ala Ile
        115                 120                 125

Leu Ser Leu Gln Tyr His Tyr Asp Ser Phe Glu Arg Leu Tyr Pro Tyr
    130                 135                 140

Val Gly Val Gly Val Gly Arg Val Leu Phe Phe Asp Lys Thr Asp Gly
145                 150                 155                 160

Ala Leu Ser Ser Phe Asp Ile Lys Asp Lys Trp Ala Pro Ala Phe Gln
                165                 170                 175

Val Gly Leu Arg Tyr Asp Leu Gly Asn Ser Trp Met Leu Asn Ser Asp
            180                 185                 190

Val Arg Tyr Ile Pro Phe Lys Thr Asp Val Thr Gly Thr Leu Gly Pro
        195                 200                 205

Val Pro Val Ser Thr Lys Ile Glu Val Asp Pro Phe Ile Leu Ser Leu
    210                 215                 220

Gly Ala Ser Tyr Val Phe
225                 230

<210>  7
<211>  230
<212>  PRT
<213>  Artificial Sequence

<220>
<223>  alkL (mod 1)

<400>  7

Val Ser Phe Ser Asn Tyr Lys Val Ile Ala Met Pro Val Leu Val Ala
1               5                   10                  15

Asn Phe Val Leu Gly Ala Ala Thr Ala Trp Ala Asn Glu Asn Tyr Pro
            20                  25                  30

Ala Lys Ser Ala Gly Tyr Asn Gln Gly Asp Trp Val Ala Ser Phe Asn
        35                  40                  45

Phe Ser Lys Val Tyr Val Gly Glu Glu Leu Gly Asp Leu Asn Val Gly
    50                  55                  60

Gly Gly Ala Leu Pro Asn Ala Asp Val Ser Ile Gly Asn Asp Thr Thr
65                  70                  75                  80

Leu Thr Phe Asp Ile Ala Tyr Phe Val Ser Ser Asn Ile Ala Val Asp
                85                  90                  95

Phe Phe Val Gly Val Pro Ala Arg Ala Lys Phe Gln Gly Glu Lys Ser
            100                 105                 110

Ile Ser Ser Leu Gly Arg Val Ser Glu Val Asp Tyr Gly Pro Ala Ile
        115                 120                 125

Leu Ser Leu Gln Tyr His Tyr Asp Ser Phe Glu Arg Leu Tyr Pro Tyr
    130                 135                 140

Val Gly Val Gly Val Gly Arg Val Leu Phe Phe Asp Lys Thr Asp Gly
145                 150                 155                 160

Ala Leu Ser Ser Phe Asp Ile Lys Asp Lys Trp Ala Pro Ala Phe Gln
                165                 170                 175

Val Gly Leu Arg Tyr Asp Leu Gly Asn Ser Trp Met Leu Asn Ser Asp
            180                 185                 190

Val Arg Tyr Ile Pro Phe Lys Thr Asp Val Thr Gly Thr Leu Gly Pro
        195                 200                 205

Val Pro Val Ser Thr Lys Ile Glu Val Asp Pro Phe Ile Leu Ser Leu

210                 215                 220

Gly Ala Ser Tyr Val Phe
225                 230

<210>  8
<211>  446
<212>  PRT
<213>  Artificial Sequence

<220>
<223>  FadL

<400>  8

Met Ser Gln Lys Thr Leu Phe Thr Lys Ser Ala Leu Ala Val Ala Val
1               5                   10                  15

Ala Leu Ile Ser Thr Gln Ala Trp Ser Ala Gly Phe Gln Leu Asn Glu
            20                  25                  30

Phe Ser Ser Ser Gly Leu Gly Arg Ala Tyr Ser Gly Glu Gly Ala Ile
        35                  40                  45

Ala Asp Asp Ala Gly Asn Val Ser Arg Asn Pro Ala Leu Ile Thr Met
    50                  55                  60

Phe Asp Arg Pro Thr Phe Ser Ala Gly Ala Val Tyr Ile Asp Pro Asp
65                  70                  75                  80

Val Asn Ile Ser Gly Thr Ser Pro Ser Gly Arg Ser Leu Lys Ala Asp
                85                  90                  95

Asn Ile Ala Pro Thr Ala Trp Val Pro Asn Met His Phe Val Ala Pro
            100                 105                 110

Ile Asn Asp Gln Phe Gly Trp Gly Ala Ser Ile Thr Ser Asn Tyr Gly
        115                 120                 125

Leu Ala Thr Glu Phe Asn Asp Thr Tyr Ala Gly Gly Ser Val Gly Gly
    130                 135                 140

Thr Thr Asp Leu Glu Thr Met Asn Leu Asn Leu Ser Gly Ala Tyr Arg
145                 150                 155                 160

Leu Asn Asn Ala Trp Ser Phe Gly Leu Gly Phe Asn Ala Val Tyr Ala
                165                 170                 175

Arg Ala Lys Ile Glu Arg Phe Ala Gly Asp Leu Gly Gln Leu Val Ala
            180                 185                 190

Gly Gln Ile Met Gln Ser Pro Ala Gly Gln Thr Gln Gln Gly Gln Ala
        195                 200                 205

Leu Ala Ala Thr Ala Asn Gly Ile Asp Ser Asn Thr Lys Ile Ala His
    210                 215                 220

Leu Asn Gly Asn Gln Trp Gly Phe Gly Trp Asn Ala Gly Ile Leu Tyr
225                 230                 235                 240

Glu Leu Asp Lys Asn Asn Arg Tyr Ala Leu Thr Tyr Arg Ser Glu Val
                245                 250                 255

Lys Ile Asp Phe Lys Gly Asn Tyr Ser Ser Asp Leu Asn Arg Ala Phe
            260                 265                 270

Asn Asn Tyr Gly Leu Pro Ile Pro Thr Ala Thr Gly Gly Ala Thr Gln
        275                 280                 285

Ser Gly Tyr Leu Thr Leu Asn Leu Pro Glu Met Trp Glu Val Ser Gly
    290                 295                 300

Tyr Asn Arg Val Asp Pro Gln Trp Ala Ile His Tyr Ser Leu Ala Tyr
305                 310                 315                 320

Thr Ser Trp Ser Gln Phe Gln Gln Leu Lys Ala Thr Ser Thr Ser Gly
                325                 330                 335

Asp Thr Leu Phe Gln Lys His Glu Gly Phe Lys Asp Ala Tyr Arg Ile
            340                 345                 350

Ala Leu Gly Thr Thr Tyr Tyr Tyr Asp Asp Asn Trp Thr Phe Arg Thr
        355                 360                 365

Gly Ile Ala Phe Asp Asp Ser Pro Val Pro Ala Gln Asn Arg Ser Ile
    370                 375                 380

Ser Ile Pro Asp Gln Asp Arg Phe Trp Leu Ser Ala Gly Thr Thr Tyr
385                 390                 395                 400

Ala Phe Asn Lys Asp Ala Ser Val Asp Val Gly Val Ser Tyr Met His
                405                 410                 415

Gly Gln Ser Val Lys Ile Asn Glu Gly Pro Tyr Gln Phe Glu Ser Glu
            420                 425                 430

Gly Lys Ala Trp Leu Phe Gly Thr Asn Phe Asn Tyr Ala Phe

435                    440                    445

## Description

### FIELD OF THE INVENTION

The present invention relates to biotechnological methods and cells for producing at least one acyl amino acid from amino acids and/or fatty acids.

### BACKGROUND OF THE INVENTION

Acyl amino acids are a class of surface-active agents with a variety of uses, for example as detergents for washing purposes, emulsifiers in food products and as essential ingredients in various personal care products such as shampoos, soaps, moisturizing agents and the like. In addition to having both hydrophobic and hydrophilic regions, a prerequisite for use as a surfactant, the compounds (surfactants) are made of naturally occurring molecules, more specifically amino acids and fatty acids, which are not only non-hazardous and environmentally acceptable but may be readily produced at a large scale using inexpensive biological raw materials. In pharmacological research, acyl amino acids are used as neuromodulators and probes for new drug targets.

Acyl amino acids have been isolated from a multitude of biological sources and are believed to have a range of functions, for example as signalling molecules in mammalian tissues, as building blocks for antibiotics in bacterial cultures, as compounds involved in bacterial protein sorting and the like.

Traditionally acyl amino acids have been produced at an industrial scale starting with materials derived from tropical oils. More specifically, activated fatty acids provided in the form of acid chlorides may be used to acylate amino acids in an aqueous alkaline medium as described in GB1483500. Shortcomings of such approaches include the need to add hazardous chemicals such as sulphuric acid or anhydrides thereof. Other synthetic approaches are associated with the accumulation of by-products such as chloride salts which have undesirable effects on surfactancy.

Thus these very effective and high-foaming surfactants are gaining significant and increasing value in the chemical industry as an ingredient in home and personal care applications. This would be especially true, if an environmentally benign production process would be available, as the current chemical production processes involve compounds such as phosgene or phosphorus trichloride. Such an environmentally friendly process would be the biocatalytic conversion of amino and fatty acids to acyl amino acids by whole-cell biocatalysts not requiring any toxic chemicals.

A range of biotechnological routes towards production of acyl amino acids has been described. However, none of them is adequate for the commercial large-scale production of acyl amino acids owing to low yields, insufficient purities and the need for multi-step purification procedures. In particular, only a small proportion of the carbon substrates fed to biotechnologically useful organisms is actually converted to the sought-after product, whilst much of it is consumed by reactions of the primary metabolism. These carbon substrates may even include amino and fatty acids which may be used by the whole-cell biocatalysts as an energy source thus reducing product yield per substrate and, in turn, increasing manufacturing costs.

### DESCRIPTION OF THE INVENTION

The present invention attempts to solve the problems above by providing at least one method of producing acyl amino acids using biocatalysts with attenuated capability to use amino acids and/or fatty acids as an energy source for cell growth and maintenance. Accordingly, the yield and purity of the product of the present invention, namely acyl amino acids with less catalysts or unwanted by-products, may be improved compared to the processes in the state of the art.

According to one aspect of the present invention there is provided a microbial cell for producing at least one acyl amino acid, wherein the cell is genetically modified to comprise:
- a first genetic mutation that enables the cell to produce at least one acyl amino acid; and
- a second genetic mutation that enables the cell to decrease glutamate breakdown relative to the wild type cell.

The cell according to any aspect of the present invention may be capable of producing acyl amino acids and the cell may further comprise a second genetic mutation that reduces the use of amino acids and/or fatty acids as a carbon/ energy source for the cell. The second genetic mutation in the cell that reduces the use of available amino acids and/or fatty acids as a carbon source may be a genetic mutation in an enzyme that is involved in glutamate breakdown. In particular, the second genetic mutation may result in a decrease in the activity of at least one enzyme involved in glutamate breakdown relative to a wild type cell. More in particular, the second genetic mutation may result in a reduced expression relative to a wild type cell of at least one enzyme that catalyses the breakdown of glutamate via at least one compound selected from the group consisting of acetyl-CoA, acetate, pyruvate, succinate, succinyl-CoA, fumarate, malate, citrate, lactate, 2-oxoglutarate, and the like to ultimately carbon dioxide, biomass or a fermentation product.

In one example, the first genetic mutation may be in at least one amino acid-N-acyl-transferase (E₁) and (ii) acyl-CoA synthetase (E₂). In another example, the first genetic mutation may result in increased activity relative to the wild type cell of glycine-N-acyl transferase (E₁ₐ) that is capable of producing N-acyl glutamate. In yet another example, the cell according to any aspect of the present invention may be genetically modified to have a mutation in an enzyme that is involved in glutamate breakdown and a mutation in an enzyme that may result in increased activity relative to the wild type cell of glycine-N-acyl transferase (E₁ₐ).

According to another aspect of the present invention, there is provided a microbial cell for producing at least one acyl amino acid, wherein the cell is genetically modified to comprise:
- a first genetic mutation that enables the cell to produce N-acyl glutamate; and
- a second genetic mutation that enables the cell to decrease glutamate breakdown relative to the wild type cell
wherein the first genetic mutation comprises at least a genetic mutation that results in increased activity of glycine-N-acyl transferase (E₁ₐ) relative to the wild type cell.

In particular, glutamate breakdown may involve the breakdown of glutamate via at least one compound selected from the group consisting of acetyl-CoA, acetate, pyruvate, succinate, succinyl-CoA, fumarate, malate, citrate, lactate, 2-oxoglutarate and the like. More in particular, the second genetic mutation may result in a reduced expression relative to a wild type cell of at least one enzyme that catalyses the breakdown of glutamate via at least one compound selected from the group consisting of acetyl-CoA, acetate, pyruvate, succinate, succinyl-CoA, fumarate, malate, citrate, lactate and 2-oxoglutarate, to ultimately carbon dioxide, biomass or a fermentation product.

As used herein the term "glutamate breakdown" may refer to the chemical processes which are part of glutamate metabolism whereby glutamate is degraded, including those steps wherein an enzyme catalyses the use of glutamate to form another material. Each step of the metabolic pathway is usually catalysed by an enzyme, whose structure is encoded by a gene. In particular, glutamate may be broken down to a variety of compounds including but not limited to acetyl-CoA, acetate, pyruvate, succinate, succinyl-CoA, fumarate, malate, citrate, lactate and 2-oxoglutarate, which are then further metabolized to carbon dioxide, a component of biomass, that is an amino acid, a protein, a phospholipid, a polysaccharide, a nucleic acid, or any other small or macromolecule being a constituent of microbial biomass, or a secreted fermentation product.

In particular, glutamate breakdown may include at least one degradation pathway selected from the following pathways: The assays that may be used to measure the activity of these enzymes are provided at least in brackets or are described in the journal articles provided in the brackets.

Degradation pathway I:
1. Glutamate + NAD(P)⁺ → 2-oxoglutarate + NAD(P)H + H⁺ + NH₄⁺ (Glutamate dehydrogenase: EC 1.4.1.2, 1.4.1.3 or 1.4.1.4) (E₁₁) (Glutamate Dehydrogenase Activity Assay Kit, Fa. Sigma Aldrich; Catalog-No.: MAK099)
2. 2-Oxoglutarate metabolization via tricarboxylic acid cycle

Degradation pathway II:
1. Glutamate + H⁺ → 4-aminobutanoate + CO₂ (Glutamate decarboxylase: EC 4.1.1.15) (E₁₂) (Yu et al. 2011)
2. 4-Aminobutanoate + 2-oxoglutarate / pyruvate → succinate semialdehyde + L-glutamate / L- alanine (4-aminobutyrate aminotransferase: EC 2.6.1.19 or EC 2.6.1.96) (E₁₃) (Liu et al. 2005, Jeffery et al. 1988)
3. Succinate semialdehyde + NAD(P)⁺ + H₂O → succinate + NAD(P)H + H⁺ (succinate-semialdehyde dehydrogenase: EC 1.2.1.16 or EC 1.2.1.24) (E₁₄) (Esser et al. 2013)
4. Succinate metabolization via tricarboxylic acid cycle

Degradation pathway III:
1. Glutamate + H⁺ → 4-aminobutanoate + CO₂ (Glutamate decarboxylase: EC 4.1.1.15) (E₁₂)
2. 4-Aminobutanoate + 2-oxoglutarate / pyruvate → succinate semialdehyde + L-glutamate / L- alanine (4-aminobutyrate aminotransferase: EC 2.6.1.19 or EC 2.6.1.96) (E₁₃)
3. Succinate semialdehyde + NAD(P)⁺ + H₂O → succinyl-CoA + NAD(P)H + H⁺ (succinate-semialdehyde dehydrogenase (acylating): EC 1.2.1.76) (E₁₅) (Söhling and Gottschalk 1993)
4. Succinyl-CoA metabolization via tricarboxylic acid cycle

Degradation pathway IV:
1. Glutamate + oxaloacetate → 2-oxoglutarate + L-aspartate (glutamate:aspartate transaminase: EC 2.6.1.1) (E₁₆) (Mavrides and Orr 1975, Karmen 1955);
2. 2-oxoglutarate metabolization via tricarboxylic acid cycle

Degradation pathway V:
1. Glutamate + NAD(P)⁺ → 2-oxoglutarate + NAD(P)H + H⁺ + NH₄⁺ (Glutamate dehydrogenase: EC 1.4.1.2, 1.4.1.3 or 1.4.1.4) (E₁₁)
2. 2-Oxoglutarate + NAD(P)H + H⁺ → 2-hydroxyglutarate + NAD(P)⁺ + H₂O (2-hydroxyglutarate dehydrogenase: EC 1.1.99.2) (E₁₇) (Kalliri et al. 2008)
3. 2-Hydroxyglutarate + acyl-CoA → 2-hydroxyglutaryl-CoA + fatty acid (2-hydroxyglutarate CoA transferase: EC 2.8.3.12) (E₁₈) (Buckel et al. 1981)
4. 2-Hydroxyglutaryl-CoA → glutaconyl-CoA + H₂O (2-hydroxyglutaryl-CoA dehydratase: EC 4.2.1.-) (E₁₉) (Parthasarathy et al. 2011)
5. Glutaconyl-CoA → crotonyl-CoA + CO₂ (glutaconyl-CoA decarboxylase: EC 4.1.1.70) (E₂₀) (Härtel et al. 1993)
6. Crotonyl-CoA + H₂O → 3-hydroxybutyryl-CoA (enoyl-CoA hydratase / 3-hydroxybutyryl-CoA dehydratase: EC 4.2.1.17, EC 4.2.1.55 or EC 4.2.1.150) (E₂₁) (Feng et al. 2002, Moskowitz and Merrick 1969)
7. 3-Hydroxybutyryl-CoA + NAD(P)⁺ → acetoacetyl-CoA + NAD(P)H + H⁺ (3-hydroxybutyryl-CoA dehydrogenase: EC 1.1.1.35 or EC 1.1.1.157) (E₂₂) (Hawkins et al. 2014, Taylor et al. 2010)
8. Acetoacetyl-CoA + CoA→Acetyl-CoA + acetate (thiolase: EC 2.3.1.9 or EC 2.3.1.16) (E₂₃) (Wiesenborn et al. 1988, Yamashita et al. 2006)
9. Acetyl-CoA and acetate metabolization via tricarboxylic acid cycle

Degradation pathway VI:
1. Glutamate → 3-methylaspartate (glutamate mutase: EC 5.4.99.1) (E₂₄) (Chen and Marsh 1997)
2. 3-Methylaspartate → mesaconate + NH₄⁺ (3-methylaspartase EC 4.3.1.2) (E₂₅) (Barker et al. 1959)
3. Mesaconate + H₂O → citramalate (citramalate hydrolyase: EC 4.2.1.34) (E₂₆) (Blair and Barker 1966, Wang and Barker 1969)
4. Citramalate → pyruvate + acetate (citramalate lyase: EC 4.1.3.22) (E₂₇) (Barker 1967)
5. Pyruvate and acetate metabolization via pyruvate dehydrogenase and tricarboxylic acid cycle

Degradation pathway VII:
1. Glutamate + pyruvate → 2-oxoglutarate + L-alanine (glutamate:pyruvate or alanine transaminase: EC 2.6.1.2) (E₂₈) (Duff et al. 2012);
2. 2-Oxoglutarate metabolization via tricarboxylic acid cycle

In one example, the cell according to any aspect of the present invention may be genetically modified to attenuate at least one degradation pathway from Degradation pathway I-VII to reduce glutamate breakdown relative to a wild type cell. In another example, the decrease in glutamate breakdown in a cell according to any aspect of the present invention may be due to attenuation in a combination of Degradation pathways I-VII. In one example, the decrease in glutamate breakdown in a cell according to any aspect of the present invention may be due to attenuation in Degradation pathway I and II, I and III; I and IV; I and V; I and VI; I and VII; II and III; II and IV; II and V; II and VI; II and VII; III and IV; III and V; III and VI; III and VII; IV and V; IV and VI; IV and VII; V and VI; V and VII; I, II and III; I, II and IV; I, II and V; I, II and VI; I, II and VII; I, III and V; I, III and VI; I, III and VII; I, IV and V; I, IV and VI; I, IV and VII; I, V and VI; I, V and VII; II, III and IV; II, III and V; II, III and VI; II, III and VII; III, IV and V; III, IV and VI; III, IV and VII; IV, V and VI; IV, V and VII; I, II, III and IV; I, II, III and V; I, II, III and VI; I, II, III and VII; II, III, IV and V; II, III, IV and VI; II, III, IV and VII; I, II, III, IV and V; I, II, III, IV and VI; I, II, III, IV and VII; I, II, III, IV, V and VI; I, II, III, IV, V and VII; I, II, III, IV, V and VI or I, II, III, IV, V, VI and VII. The cell according to any aspect of the present invention may comprise at least one genetic mutation in any of the enzymes mentioned above involved in any one of the Degradation pathways I-VII to result in attenuation in glutamate breakdown. In one example there may be a genetic mutation in a combination of enzymes that are part of any one of the Degradation pathways I-VII that may result in a decrease in glutamate breakdown in a cell according to any aspect of the present invention relative to a wild type cell.

In another example, a cell according to any aspect of the present invention may comprise a second genetic mutation in at least one of the enzymes E₁₁- E₂₈ that may result in an overall decrease in glutamate breakdown relative to the wild type cell. In particular, the second genetic mutation may comprise a genetic mutation in a combination of enzymes that may be involved in the same or different pathways in glutamate breakdown. The final result will be a cell according to any aspect of the present invention with decreased glutamate breakdown thus higher concentration of glutamate. As used herein the term "glutamate" refers to the amino acid α-glutamate or α-glutamic acid.). A genetic mutation that decreases glutamate breakdown includes the inactivation of a gene which encodes for part of the glutamate metabolic pathway as shown above. Accordingly, inactivating at least one of the enzymes E₁₁- E₂₈ may result in a decrease in glutamate breakdown in the cell according to any aspect of the present invention relative to a wild type cell.

As used herein the term " tricarboxylic acid cycle (TCA cycle)" may also refer to the citric acid cycle or the Krebs cycle. In particular, the TCA cycle is a series of chemical reactions used by all aerobic organisms to generate energy through the oxidation of acetate derived from carbohydrates, fats and proteins into carbon dioxide and chemical energy in the form of adenosine triphosphate (ATP). In addition, the cycle provides precursors of certain amino acids as well as the reducing agents NADH and NADPH that is used in numerous other biochemical reactions. The name of this metabolic pathway is derived from citric acid (a type of tricarboxylic acid) that is consumed and then regenerated by this sequence of reactions to complete the cycle. In addition, the cycle consumes acetate (in the form of acetyl-CoA) and water, reduces NAD(P)+ to NAD(P)H, and produces carbon dioxide as a waste byproduct. The NADH generated by the TCA cycle is fed into the oxidative phosphorylation (electron transport) pathway. The net result of these two closely linked pathways is the oxidation of nutrients to produce usable chemical energy in the form of ATP.

The cell according to any aspect of the present invention may comprise a genetic mutation that results in decreased glutamate breakdown wherein the mutation is in at least one enzyme selected from at least one of the group of enzymes consisting of:
(i) E₁₁;
(ii) E₁₂, E₁₃, and E₁₄;
(iii) E₁₂, E₁₃, and E₁₅;
(iv) E₁₆;
(v) E₁₁, E₁₇, E₁₈, E₁₉, E₂₀, E₂₁, E₂₂, and E₂₃;
(vi) E₂₄, E₂₅, E₂₆, and E₂₇; and
(vii) E₂₈.

In one example, the mutation may be in E₁₁. In another example, the mutation may be in E₁₂. In yet another example, the mutation may be in E₁₆ or in E₁₇ or in E₂₄ or in E₂₈. In a further example, the mutation may be in E₁₃ and E₁₄. In yet a further example, the mutation may be in at least one enzyme selected from E₁₁, and E₁₂- E₂₈. The mutation that may result in reduced glutamate breakdown in the cell according to any aspect of the present invention may be in a combination of enzymes that may be part of a single pathway or multiple pathways that are part of glutamate breakdown. For example, the mutation may be in E₁₁ and at least one enzyme selected from the group consisting of E₁₂- E₂₈.

According to any aspect of the present invention, the first genetic mutation may result in the cell having increased expression of (i) an amino acid-N-acyl-transferase (E₁) that is capable of producing N-acyl glutamate and (ii) acyl-CoA synthetase (E₂). The combination of amino acid-N-acyl transferase and an acyl-CoA synthetase, expressed according to any aspect of the present invention may be used to convert a variety of fatty acids and/or amino acids including a mixture comprising unsaturated and saturated fatty acids, to acyl amino acids. In particular, the amino acid-N-acyl transferases may be used to convert short unsaturated fatty acids such as lauroleic acid to an acyl amino acid. More in particular, the amino acid-N-acyl-transferase may be capable of converting a variety of fatty acids including short unsaturated fatty acids such as lauroleic acid to an acyl amino acid with an increase in the yields of acyl amino acids produced. Further, the composition of acyl amino acids produced in a cell, more specifically the length of fatty acids incorporated into such acyl amino acids, may be controlled by introducing into the cell one or more specific acyl-CoA thioesterases or altering the expression of one or more acyl-CoA thioesterases endogenously expressed by the cell. In particular, the first genetic mutation may result in the cell having increased expression of (i) glycine-N-acyl-transferase (E₁ₐ) and (ii) acyl-CoA synthetase (E₂).

The term "amino acid-N-acyl transferase", as used herein, refers to an enzyme capable of catalysing the conversion of acyl-CoA, for example the CoA ester of an acid, and an amino acid, for example a proteinogenic amino acid, for example glutamic acid, to an acyl amino acid. More in particular, the term "glycine N-acyl transferase" refers to an amino acid-N-acyl transferase that is capable of producing N-acyl glutamate alone and/or in combination with another acyl amino acid. In one example, the glycine N-acyl transferase used according to any aspect of the present invention may produce N-acyl glutamate only. In another example, the glycine N-acyl transferase used according to any aspect of the present invention may produce N-acyl glutamate and N-acyl glycinate. More in particular, the glycine N-acyl transferase used according to any aspect of the present invention may always produce at least N-acyl glutamate. Suitable amino acid-N-acyl transferases have been described in the prior art, for example in Waluk, D. P., Schultz, N., and Hunt, M. C. (2010). In particular, the amino acid sequence of glycine N-acyl transferase (E₁ₐ) used according to any aspect of the present invention may be selected from the sequences SEQ ID NO: 4, NP_001010904.1,NP_659453.3, XP_001147054.1, AAH16789.1, AA073139.1, XP_003275392.1, XP_002755356.1, XP_003920208.1, XP_004051278.1, XP_006147456.1, XP_006214970.1, XP_003801413.1, XP_006189704.1, XP_003993512.1, XP_005862181.1, XP_007092708.1, XP_006772167.1, XP_006091892.1, XP_005660936.1, XP_005911029.1, NP_001178259.1, XP_004016547.1, XP_005954684.1, ELR45061.1, XP_005690354.1, XP_004409352.1, XP_007519553.1, XP_004777729.1, XP_005660935.1, XP_004824058.1, XP_006068141.1, XP_006900486.1, XP_007497585.1, XP_002821801.2, XP_007497583.1, XP_003774260.1, XP_001377648.2, XP_003909843.1, XP_003801448.1, XP_001091958.1, XP_002821798.1, XP_005577840.1, XP_001092197.1, NP_001207423.1, NP_001207425.1, XP_003954287.1, NP_001271595.1, XP_003909848.1, XP_004087850.1, XP_004051279.1, XP_003920209.1, XP_005577835.1, XP_003774402.1, XP_003909846.1, XP_004389401.1, XP_002821802.1, XP_003774401.1, XP_007497581.1, EHH21814.1, XP_003909845.1, XP_005577839.1, XP_003774403.1, XP_001092427.1, XP_003275395.2, NP_542392.2, XP_001147271.1, XP_005577837.1, XP_003826420.1, XP_004051281.1, XP_001147649.2, XP_003826678.1, XP_003909847.1, XP_004682812.1, XP_004682811.1, XP_003734315.1, XP_004715052.1, BAG62195.1, XP_003777804.1, XP_003909849.1, XP_001092316.2, XP_006167891.1, XP_540580.2, XP_001512426.1, EAW73833.1, XP_003464217.1, XP_007519551.1, XP_003774037.1, XP_005954680.1, XP_003801411.1, NP_803479.1, XP_004437460.1, XP_006875830.1, XP_004328969.1, XP_004264206.1, XP_004683490.1, XP_004777683.1, XP_005954681.1, XP_003480745.1, XP_004777682.1, XP_004878093.1, XP_007519550.1, XP_003421399.1, EHH53167.1, XP_006172214.1 ,XP_003993453.1 ,AAI12537.1, XP_006189705.1 ,Q2KIR7.2, XP_003421465.1, NP_001009648.1, XP_003464328.1, XP_001504745.1, ELV11036.1, XP_005690351.1, XP_005216632.1, EPY77465.1, XP_005690352.1, XP_004016544.1, XP_001498276.2, XP_004264205.1, XP_005690353.1, XP_005954683.1, XP_004667759.1, XP_004479306.1, XP_004645843.1, XP_004016543.1, XP_002928268.1, XP_006091904.1, XP_005331614.1, XP_007196549.1, XP_007092705.1, XP_004620532.1, XP_004869789.1, EHA98800.1, XP_004016545.1, XP_004479307.1, XP_004093105.1, NP_001095518.1, XP_005408101.1, XP_004409350.1, XP_001498290.1, XP_006056693.1, XP_005216639.1, XP_007455745.1, XP_005352049.1, XP_004328970.1, XP_002709220.1, XP_004878092.1, XP_007196553.1, XP_006996816.1, XP_005331615.1, XP_006772157.1, XP_007196552.1, XP_004016546.1, XP_007628721.1, NP_803452.1, XP_004479304.1, DAA21601.1, XP_003920207.1, XP_006091906.1, XP_003464227.1, XP_006091903.1, XP_006189706.1, XP_007455744.1, XP_004585544.1, XP_003801410.1, XP_007124812.1, XP_006900488.1, XP_004777680.1, XP_005907436.1, XP_004389356.1, XP_007124811.1, XP_005660937.1, XP_007628724.1, XP_003513512.1, XP_004437813.1, XP_007628723.1, ERE78858.1, EPQ15380.1, XP_005862178.1, XP_005878672.1, XP_540581.1, XP_002928267.1, XP_004645845.1, EPQ05184.1, XP_003513511.1, XP_006214972.1, XP_007196545.1, XP_007196547.1, XP_006772160.1, XP_003801409.1, NP_001119750.1, XP_003801412.1, XP_006772159.1, EAW73832.1, XP_006091897.1, XP_006772163.1, XP_006091898.1, XP_005408105.1, XP_006900487.1, XP_003993454.1, XP_003122754.3, XP_007455746.1, XP_005331618.1, XP_004585337.1, XP_005063305.1, XP_006091895.1, XP_006772156.1, XP_004051276.1, XP_004683488.1, NP_666047.1, NP_001013784.2, XP_006996815.1, XP_006996821.1, XP_006091893.1, XP_006173036.1, XP_006214971.1, EPY89845.1, XP_003826423.1, NP_964011.2, XP_007092707.1, XP_005063858.1, BAL43174.1, XP_001161154.2, XP_007124813.1, NP_083826.1XP_003464239.1, XP_003275394.1, ELK23978.1, XP_004878097.1, XP_004878098.1, XP_004437459.1, XP_004264204.1, XP_004409351.1, XP_005352047.1, Q5RFP0.1, XP_005408107.1, XP_007659164.1, XP_003909852.1, XP_002755355.1, NP_001126806.1, AAP92593.1, NP_001244199.1, BAA34427.1, XP_005063859.1, NP_599157.2, XP_004667761.1, XP_006900489.1, XP_006215013.1, XP_005408100.1, XP_007628718.1, XP_003514769.1, XP_006160935.1, XP_004683489.1, XP_003464329.1, XP_004921258.1, XP_003801447.1, XP_006167892.1, XP_004921305.1, AAH89619.1, XP_004706162.1, XP_003583243.1, EFB16804.1, XP_006728603.1, EPQ05185.1, XP_002709040.1, XP_006875861.1, XP005408103.1, XP_004391425.1, EDL41477.1, XP_006772158.1, EGW06527.1, AAH15294.1, XP_006772162.1, XP_005660939.1, XP_005352050.1, XP_006091901.1, XP_005878675.1, XP_004051323.1, EHA98803.1, XP_003779925.1, EDM12924.1, XP_003421400.1, XP_006160939.1, XP_006160938.1, XP_006160937.1, XP_006160936.1, XP_005702185.1, XP_005313023.1, XP_003769190.1, XP_002714424.1, XP_004715051.1, XP_007661593.1, XP_004590594.1, ELK23975.1, XP_004674085.1, XP_004780477.1, XP_006231186.1, XP_003803573.1, XP_004803176.1, EFB16803.1, XP_006056694.1, XP_005441626.1, XP_005318647.1XP_004605904.1, XP_005862182.1, XP_003430682.1, XP_004780478.1, XP_005239278.1, XP_003897760.1, XP_007484121.1, XP_004892683.1, XP_004414286.1, XP_006927013.1, XP_003923145.1, XP_852587.2, AAP97178.1, EHH53105.1, XP_005408113.1, XP_002915474.1, XP_005377590.1, XP_527404.2, XP_005552830.1, XP_004044211.1, NP_001180996.1, XP_003513513.2, XP_001498599.2, XP_002746654.1, XP_005072349.1, XP_006149181.1, EAX04334.1, XP_003833230.1, XP_005216635.1, XP_003404197.1, XP_007523363.1, XP_007433902.1, XP_003254235.1, XP_004471242.1, XP_005216634.1, XP_006860675.1, XP_004771956.1, XP_006038833.1, NP_001138534.1, XP_007068532.1, XP_003510714.1, ERE87950.1, XP_003986313.1, XP_006728644.1, XP_004878099.1, XP_003468014.1, XP_007095614.1, XP_004648849.1, XP_004869795.1, XP_004018927.1, XP_005696454.1, XP_006201985.1, XP_005960697.1, XP_004813725.1, XP_005496926.1, ELR45088.1, XP_004696625.1, XP_005860982.1, XP_005911003.1, XP_006260162.1, EPQ04414.1, XP_006099775.1, NP_001138532.1, XP_006190795.1, XP_004649775.1, XP_004424497.1, XP_004390885.1, XP_005911004.1, XP_003777803.1, XP_004312259.1, XP_005529140.1, XP_005314582.1, XP_006926523.1, XP_006926522.1, XP_004683491.1, XP_003826680.1, XP_003215018.1, XP_003215087.1 EGW12611.1, XP_006113023.1, XP_006882182.1, XP_007425200.1, XP_006041342.1, NP_001138533.1, EMP27694.1, XP_007497753.1, XP_006034252.1, or a variant thereof. Throughout this disclosure, any data base code, unless specified to the contrary, refers to a sequence available from the NCBI data bases, more specifically the version online on 5^{th} August 2013, and comprises, if such sequence is a nucleotide sequence, the polypeptide sequence obtained by translating the former.

In particular, the first genetic mutation may increase the activity of glycine N-acyl transferase (E₁ₐ) and acyl-CoA synthetase (E₂) relative to the wild type cell. More in particular, the sequence of glycine N-acyl transferase (E₁ₐ) used according to any aspect of the present invention may have 50%, 55%, 60%, 65%, 70%, 75%, 80%, 85%, 90%, 95%, 100% sequence identity with SEQ ID NO:4 and/or the sequence of acyl-CoA synthetase (E₂) used according to any aspect of the present invention may have 50%, 55%, 60%, 65%, 70%, 75%, 80%, 85%, 90%, 95%, 100% sequence identity with SEQ ID NO:1.

The cell according to any aspect of the present invention may comprise a genetic mutation in a second amino acid acyl transferase that may be capable of working on other amino acids or variants thereof related to the breakdown of glutamic acid and/or glutamate. In one example, the other amino acids or variants thereof may be selected from the group consisting of proline, arginine, glutathione, ornithine, spermidine, spermine, sarcosine and putrescine and sodium lauroyl sarcosinate. In another example, there may be more than 1, 2, 3, 4, 5, 6, 7 or the like mutations in the cell according to any aspect of the present invention that may result in the cell expressing more than one amino acid N-acyltransferase (E₁) besides glycine-N-acyl transferase (E₁ₐ). In this example, the cell according to any aspect of the present invention may be capable of expressing an amino acid N-acyl-transferase, glycine-N-acyl transferase (E₁ₐ) and acyl-CoA synthetase (E₂). This cell may then be capable of producing N-acyl glutamate and other relevant N-acyl amino acids depending on the amino acid-N-acyl- transferase expressed and the amino acid used as the substrate. For example, the cell according to any aspect of the present invention may be genetically modified in the following manner:
- First genetic mutation in glycine-N-acyl transferase (E₁ₐ) and acyl-CoA synthetase (E₂),
- Second genetic mutation in at least one enzyme that results in a decrease in activity relative to a wild type cell of at least one enzyme involved in glutamate breakdown,
- Further genetic mutation in a second amino acid-N-acyl- transferase (E₁) and
- Any further genetic mutation as mentioned below.

Glycine-N-acyl transferase (E₁ₐ) may catalyse the following reaction: glutamate + acyl-CoA → acylglutamate + CoA. More in particular, the cell according to any aspect of the present invention comprises a first genetic mutation that increases the activity of glycine-N-acyl transferase (E₁ₐ) and acyl-CoA synthetase (E₂) relative to the wild type cell and a second genetic mutation that decreases the activity of at least one enzyme involved in glutamate breakdown relative to a wild type cell. Even more in particular, the enzyme involved in glutamate breakdown may be selected from the group consisting of E₁₁- E₂₈. The second genetic mutation in at least one of the enzymes E₁₁- E₂₈ may result in an overall decrease in glutamate breakdown relative to the wild type cell. In particular, the second genetic mutation may comprise a genetic mutation in a combination of enzymes that may be involved in the same or different pathways in glutamate breakdown. The final result will be a cell according to any aspect of the present invention with decreased glutamate breakdown thus higher concentration of glutamate.

The acyl-CoA substrate consumed in the cell according to any aspect of the present invention may be purified from a cell, chemically synthesised or produced using an acyl-CoA synthetase. The term "acyl-CoA synthetase", as used herein, refers to an enzyme capable of catalysing the ATP- or GTP-dependent conversion of a fatty acid and CoA or ACP to acyl-CoA or acyl-ACP. In one example, the acyl-CoA synthetase may comprise nucleotide sequence SEQ ID NO:2 or amino acid sequence SEQ ID NO:1.

According to any aspect of the present invention, the term 'acyl-CoA synthetase' may refer to an acyl-CoA/ACP synthetase that may be capable of producing acyl thioester, i.e. acyl-CoA or acyl-ACP and/or catalysing the following reaction:

fatty acid + CoA/ACP + ATP/GTP → acyl-CoA/ACP + ADP/GDP + Pi

Examples of acyl-CoA synthetases may include EC 6.2.1.3, EC 6.2.1.10, EC 6.2.1.15, EC 6.2.1.20 and the like. The state of the art describes various methods to detect acyl-CoA synthetase activity. For example, the activity of an acyl-CoA synthetase may be assayed as follows: the standard reaction mixture for the spectrophotometric assay (total volume, 1 ml) is composed of 0.1 M Tris-HC1 buffer pH 8.0, 1.6 mM Triton X-100, 5 mM dithiothreitol, 0.15 M KCI, 15 mM MgCl₂, 10 mM ATP, 0.1 mM potassium palmitate, 0.6 mM CoA, 0.2 mM potassium phosphoenolpyruvate, 0.15 mM NADH, 45 pg adenylate kinase per mL, 30 pg pyruvate kinase per mL and 30 pg lactate dehydrogenase per mL. The oxidation of NADH at 334 nm is followed with a recording spectrophotometer.

Alternatively, the activity of an acyl-CoA synthetase may be assayed as described in the state of the art, for example Kang, Y., et al, 2010. Briefly, the amount of free thiol in the form of unreacted CoASH may be determined by adding Ellmann's reagent and spectrophotometrically monitoring the absorbance at 410 nm, in a reaction buffer comprising 150 mM Tris-HCl (pH 7.2), 10 mM MgCl₂, 2 mM EDTA, 0.1 % Triton X-100, 5 mM ATP, 0.5 mM Coenzyme A (CoASH) and a fatty acid (30 to 300 mM).

Various acyl-CoA synthetases have been described in the state of the art, for example YP_001724804.1, WP_001563489.1 and NP_707317.1. In one example, the acyl-CoA synthetase comprises SEQ ID NO: 1 or YP_001724804.1 or a variant thereof.

The cell according to any aspect of the present invention may be genetically different from the wild type cell. The genetic difference between the cell according to any aspect of the present invention and the wild type cell may be in the presence of a complete gene, amino acid, nucleotide etc. in the cell according to any aspect of the present invention that may be absent in the wild type cell. In one example, the cell according to any aspect of the present invention may comprise enzymes that enable the cell to produce at least one acyl amino acid and cell may further comprise at least one mutation that results in decreased glutamate breakdown. The wild type cell relative to the cell according to any aspect of the present invention may have none or no detectable activity of the enzymes that enable the cell according to any aspect of the present invention to produce at least one acyl amino acid; and the wild type cell may be capable of metabolising glutamate as per normal.

The phrase "wild type" as used herein in conjunction with a cell may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell. According to any aspect of the present invention, the cell may be genetically modified so that in a defined time interval, within 2 hours, in particular within 8 hours or 24 hours, it forms at least twice, especially at least 10 times, at least 100 times, at least 1000 times or at least 10000 times more acyl amino acids than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (acyl amino acid) in the nutrient medium.

The phrase 'decreased activity of an enzyme' and like may refer to a genetic modification that may be present in the cell according to any aspect of the present invention to decrease a specific enzymatic activity and this may be done by a gene disruption or a genetic modification. In particular, the decrease in activity of an enzyme relative to the wild type cell may be a 10, 15, 20, 25, 30, 35, 40, 45, 50, 55, 60, 65, 70, 75, 80, 85, 90, 95 or 100% less than the wild type cell.

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter, increasing translation by improved ribosome binding sites or optimized codon usage or employing a gene or allele that code for a corresponding enzyme with increased activity and optionally by combining these measures. Genetically modified cells used according to any aspect of the present invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extra-chromosomally replicating vector. In particular, an increase in an activity of an enzyme relative to the wild type cell may be a 10, 15, 20, 25, 30, 35, 40, 45, 50, 55, 60, 65, 70, 75, 80, 85, 90, 95 or 100% more than the wild type cell.

In one example, the cell according to any aspect of the present invention may comprise a first genetic mutation that results in the overexpression of at least the enzymes amino acid-N-acyl transferase and acyl-CoA synthetase. In particular, the cell may over express enzymes glycine N-acyl transferase and acyl-CoA/ACP synthetase. The expression of glycine N-acyl transferase may be measured using the assay disclosed in Badenhorst CP, 2012. Namely, DTNB, water and cell lysate are mixed together and incubated at 37°C for 10 min while monitoring absorbance at 412 nm. The 412 reading may be stable before adding in glutamate and C8-CoA (pre-mixed together) to initiate the reaction. The increase in absorbance at 412 nm is followed and the enzyme activity is calculated using ε₄₁₂= 14.15 mM⁻¹cm⁻¹

In particular, the amino acid-N-acyl-transferase (E₁) may be a human glycine N-acyl-transferase (E₁ₐ). In one example, E₁ may be SEQ ID NO:5 or a variant thereof. In another example, E₁ₐ may comprise SEQ ID NO:4 and the acyl-CoA synthetase (E₂) may comprise SEQ ID NO: 1 or a variant thereof. More in particular, the cell according to any aspect of the present invention may comprise a first genetic mutation in E₁ₐ and E₂, wherein E₁ₐ may comprise a nucleic acid sequence of SEQ ID NO: 5 and E₂ may comprise nucleic acid sequence of SEQ ID NO: 2.

According to any aspect of the present invention, a formula referring to a chemical group that represents the dissociated or undissociated state of a compound capable of dissociating in an aqueous solution comprises both the dissociated and the undissociated state and the various salt forms of the group. For example, the residue -COOH comprises both the protonated (-COOH) as well as the unprotonated (-COO⁻) carboxylic acid.

The term "acyl amino acid", as used herein, refers to the product of the reaction catalysed by an amino acid-N-acyl transferase, a compound represented by the formula acyl-CO-NH-CHR-COOH, wherein R is the side chain of a proteinogenic amino acid, and wherein the term "acyl" refers to the acyl residue of a fatty acid. In one example, the term "fatty acid", as used herein, means a carboxylic acid, for example an alkanoic acid, with at least 6, 8, 10, or 12 carbon atoms. In one example, it is a linear fatty acid, in another example, it is branched. In one example it is a saturated fatty acid. In another example, it is unsaturated. In one example, it is a fatty acid with 6, 7, 8, 9, 10, 11, 12, 13, 14, 15, 16, 17, 18, 19, 20, 21, 22, 23, 24, 25, 26, 27, 28, 29, 30 carbon atoms. In particular, the acyl amino acid may be N-acyl glutamate.

The teachings of the present invention may not only be carried out using biological macromolecules having the exact amino acid or nucleic acid sequences referred to in this application explicitly, for example by name or accession number, or implicitly, but also using variants of such sequences. The term "variant", as used herein, comprises amino acid or nucleic acid sequences, respectively, that are at least 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner to the effect that the biological activity of the reference sequence or a molecule derived therefrom is preserved. The state of the art comprises algorithms that may be used to align two given nucleic acid or amino acid sequences and to calculate the degree of identity, see Arthur Lesk (2008), Thompson *et al.,* 1994, and Katoh *et al.,* 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In one example, the term "variant", with regard to amino acid sequence, comprises, in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In one example, the term "variant" of an amino acid sequence or nucleic acid sequence comprises, in addition to the above degree of sequence identity, any active portion and/or fragment of the amino acid sequence or nucleic acid sequence, respectively, or any nucleic acid sequence encoding an active portion and/or fragment of an amino acid sequence. The term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease may be capable of hydrolysing peptide bonds in polypeptides. The phrase "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, 2, or 1 order of magnitude of the values displayed by the reference molecule with respect to a specific substrate. Similarly, the term "variant" of a nucleic acid comprises nucleic acids the complementary strand of which hybridises, preferably under stringent conditions, to the reference or wild type nucleic acid. A skilled person would be able to easily determine the amino acid-N-acyl-transferases that will be capable of making proteinogenic amino acids and/or fatty acids. In particular, the variants may include but are not limited to an amino acid-N-acyl-transferase selected from the group of organisms consisting of *Nomascus leucogenys* (NI, XP_003275392.1), *Saimiri boliviensis* (Sb, XP_003920208.1 *Felis catus* (Fc, XP_003993512.1), *Bos taurus* (Bt, NP_001178259.1), *Mus musculus* (Mm, NP_666047.1) and the like.

Stringency of hybridisation reactions is readily determinable by one ordinary skilled in the art, and generally is an empirical calculation dependent on probe length, washing temperature and salt concentration. In general, longer probes require higher temperatures for proper annealing, while shorter probes need lower temperatures. Hybridisation generally depends on the ability of denatured DNA to reanneal to complementary strands when present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995). The person skilled in the art may follow the instructions given in the manual "The DIG System Users Guide for Filter Hybridization", Boehringer Mannheim GmbH, Mannheim, Germany, 1993 and in Liebl *et al.,* 1991 on how to identify DNA sequences by means of hybridisation. In one example, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example by lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C - 68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In a particularly preferred embodiment, the temperature is approximately 64 °C - 68 °C or approximately 66 °C - 68 °C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. The term "homologue" of a nucleic acid sequence, as used herein, refers to any nucleic acid sequence that encodes the same amino acid sequence as the reference nucleic acid sequence, in line with the degeneracy of the genetic code.

The cell according to any aspect of the present invention may comprise a further mutation in at least one enzyme selected from the group consisting of:
(i) An enzyme (E₃) capable of uptake of glutamate;
(ii) An enzyme (E₄) capable of interconverting acyl-CoAs and acyl-ACPs; and
(iii) An enzyme (E₅) capable of uptake of at least one fatty acid.

In particular, E₃ may be a glutamate-translocating ABC transporter or permease; E₄ may be an acyl-CoA:ACP transacylase; and E₅ may be AlkL and/or FadL. AlkL and/or FadL may function as at least one transporter protein compared to the wild type cell. In one example, the cell may be genetically modified to overexpress both the fadL and the alkL gene. In particular, the alkL may comprise a sequence that may have 50%, 55%, 60%, 65%, 70%, 75%, 80%, 85%, 90%, 95%, 100% sequence identity with SEQ ID NO:6 or 7. In particular, the fadL may comprise a sequence that may have 50%, 55%, 60%, 65%, 70%, 75%, 80%, 85%, 90%, 95%, 100% sequence identity with SEQ ID NO:8.

The cell according to any aspect of the present invention may have reduced capacity of fatty acid degradation by beta-oxidation relative to the wild type cell. In particular, the reduced fatty acid degradation activity compared to the wildtype cell may be a result of decreased expression relative to the wild type cell of at least one enzyme selected from the group consisting of acyl-CoA dehydrogenase (FadE) (E₆), enoyl-CoA hydratase (FadB) (E₇), (R)-3-hydroxyacyl-CoA dehydrogenase (FadB) (E₈) and 3-ketoacyl-CoA thiolase (FadA) (E₉).

The term "having a reduced fatty acid degradation capacity", as used herein, means that the respective cell degrades fatty acids, in particular those taken up from the environment, at a lower rate than a comparable cell or wild type cell having normal fatty acid degradation capacity would under identical conditions. In one example, the fatty acid degradation of such a cell is lower on account of deletion, inhibition or inactivation of at least one gene encoding an enzyme involved in the β-oxidation pathway. In one example, at least one enzyme involved in the β-oxidation pathway has lost, in order of increasing preference, 5, 10, 20, 40, 50, 75, 90 or 99 % activity relative to the activity of the same enzyme under comparable conditions in the respective wild type microorganism. The person skilled in the art may be familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Fujita, Y., et al, 2007). The phrase "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. It would be within the routine skills of the person skilled in the art to measure the activity of enzymes expressed by living cells using standard essays as described in enzymology text books, for example Cornish-Bowden, 1995.

Degradation of fatty acids is accomplished by a sequence of enzymatically catalysed reactions. First of all, fatty acids are taken up and translocated across the cell membrane *via* a transport/acyl-activation mechanism involving at least one outer membrane protein and one inner membrane-associated protein which has fatty acid-CoA ligase activity, referred to in the case of *E. coli* as FadL and FadD / FadK, respectively. Inside the cell, the fatty acid to be degraded is subjected to enzymes catalysing other reactions of the β-oxidation pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed as described in the state of art, for example by monitoring the concentration of NADH spectrophotometrically at 340 nm in 100 mM MOPS, pH 7.4, 0.2 mM Enoyl-CoA, 0.4 mM NADH. The resulting enoyl-CoA is converted to 3-ketoacyl-CoA *via* 3-hydroxyacyl-CoA through hydration and oxidation, catalysed by enoyl-CoA hydratase/(R)-3-hydroxyacyl-CoA dehydrogenase, referred to as FadB and FadJ in *E. coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and Fadl in *E. coli,* catalyses the cleavage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., et al, 1997.

The phrase "a cell having a reduced fatty acid degradation capacity", as used herein, refers to a cell having a reduced capability of taking up and/or degrading fatty acids, particularly those having at least eight carbon chains. The fatty acid degradation capacity of a cell may be reduced in various ways. In particular, the cell according to any aspect of the present invention has, compared to its wild type, a reduced activity of an enzyme involved in the β-oxidation pathway. The term "enzyme involved in the β-oxidation pathway", as used herein, refers to an enzyme that interacts directly with a fatty acid or a derivative thereof formed as part of the degradation of the fatty acid via the β-oxidation pathway. The β-oxidation pathway comprises a sequence of reactions effecting the conversion of a fatty acid to acetyl-CoA and the CoA ester of the shortened fatty acid. The enzyme involved in the β-oxidation pathway may by recognizing the fatty acid or derivative thereof as a substrate, converts it to a metabolite formed as a part of the β-oxidation pathway. For example, the acyl-CoA dehydrogenase is an enzyme involved in the β-oxidation pathway as it interacts with fatty acid-CoA and converts fatty acid-CoA ester to enoyl-CoA, which is a metabolite formed as part of the β-oxidation. In another example, the term "enzyme involved in the β-oxidation pathway", as used herein, comprises any polypeptide from the group comprising acyl-CoA dehydrogenase, enoyl-CoA hydratase, 3-hydroxyacyl-CoA dehydrogenase and 3-keto-acyl-CoA thiolase. The acyl-CoA synthetase may catalyse the conversion of a fatty acid to the CoA ester of a fatty acid, i.e. a molecule, wherein the functional group -OH of the carboxy group is replaced with-S-CoA and introducing the fatty acid into the β-oxidation pathway. For example, the polypeptides FadD and FadK in *E. coli* (accession number: BAA15609.1 and NP_416216.4, respectively) are acyl-CoA synthetases. In one example, the term "acyl-CoA dehydrogenase", as used herein, may be a polypeptide capable of catalysing the conversion of an acyl-CoA to enoyl-CoA, as part of the β-oxidation pathway. For example, the polypeptide FadE in *E. coli* (accession number: BAA77891.2) may be an acyl-CoA dehydrogenase. The term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, as part of the β-oxidation pathway. For example, the polypeptides FadB and FadJ in *E. coli* (accession numbers: BAE77457.1 and P77399.1, respectively) are enoyl-CoA hydratases. The term "ketoacyl-CoA thiolase", as used herein, may refer to a polypeptide capable of catalysing the cleaving of 3-ketoacyl-CoA, resulting in an acyl-CoA shortened by two carbon atoms and acetyl-CoA, as the final step of the β-oxidation pathway. For example, the polypeptides FadA and Fadl in *E. coli* (accession number: YP_491599.1and P76503.1, respectively) are ketoacyl-CoA thiolases.

Any of the enzymes used according to any aspect of the present invention, may be an isolated enzyme. In particular, the enzymes used according to any aspect of the present invention may be used in an active state and in the presence of all cofactors, substrates, auxiliary and/or activating polypeptides or factors essential for its activity. The term "isolated", as used herein, means that the enzyme of interest is enriched compared to the cell in which it occurs naturally. The enzyme may be enriched by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

The enzyme used according to any aspect of the present invention may be recombinant. The term "recombinant" as used herein, refers to a molecule or is encoded by such a molecule, particularly a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme used according to any aspect of the present invention, is recombinant or not has not necessarily implications for the level of its expression. However, in one example one or more recombinant nucleic acid molecules, polypeptides or enzymes used according to any aspect of the present invention may be overexpressed. The term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide. Genetic modifications may be directed to transcriptional, translational, and/or post-translational modifications that result in a change of enzyme activity and/or selectivity under selected and/or identified culture conditions. Thus, in various examples of the present invention, to function more efficiently, a microorganism may comprise one or more gene deletions. Gene deletions may be accomplished by mutational gene deletion approaches, and/or starting with a mutant strain having reduced or no expression of one or more of these enzymes, and/or other methods known to those skilled in the art.

The cell according to any aspect of the present invention may refer to a wide range of microbial cells. In particular, the cell may be selected from the group consisting of *Pseudomonas, Corynebacterium, Bacillus* and *Escherichia.* In one example, the cell may be *Escherichia coli.* In another example, the cell may be a lower eukaryote, such as a fungus from the group comprising *Saccharomyces, Candida, Pichia, Schizosaccharomyces* and *Yarrowia,* particularly, *Saccharomyces cerevisiae.* The microorganism may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989).

The cell according to any aspect of the present invention may be capable of producing acyl amino acids and may have reduced glutamate breakdown. In one example, the cell may be capable of producing acyl amino acids due to an increased expression of glycine-N-acyl transferase. In particular, the cell may further be capable of making N-acyl glutamate. In one example, the cell may also be capable of reduced fatty acid degradation.

The cell according to any aspect of the present invention may thus be genetically modified to:
- increase the expression relative to the wild type cell of an amino acid-N-acyl-transferase and an acyl-CoA synthetase, and
- decrease the expression relative to the wild type cell of an enzyme associated to glutamate breakdown, and/or
- increase the expression relative to the wild type cell of an enzyme associated to production of proteinogenic amino acids and/or fatty acids from at least one carbohydrate.

The term "proteinogenic amino acid", as used herein, refers to an amino acid selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Proteinogenic amino acids and fatty acids are synthesized as part of the primary metabolism of many wild type cells in reactions and using enzymes that have been described in detail in biochemistry textbooks, for example Jeremy M Berg, et al., 2002. In particular, the proteinogenic amino acid may be selected from the group consisting of glycine, glutamine, glutamate, asparagine and alanine. More in particular, the proteinogenic amino acid may be glutamate.

The cell according to any aspect of the present invention may comprise at least one genetic mutation that enables the cell to produce at least one fatty acid according to any method known in the art. In one example, the genetic mutation may enable the cell to produce at least one fatty acid by means of a malonyl-CoA dependent and malonyl-ACP independent fatty acyl-CoA metabolic pathway. This is further described in US20140051136.

The cell according to any aspect of the present invention may comprise a further genetic mutation that enables the cell to have increased expression of acyl-CoA thioesterase (E₁₀). The term "acyl-CoA thioesterase", as used herein, refers to an enzyme capable of hydrolysing acyl-CoA. In particular, the acyl-CoA thioesterase comprises a sequence selected from the group consisting of AEM72521.1 and AAC49180.1 or a variant thereof. In one example, the amino acid sequence of E₁₀ may comprise SEQ ID NO:3. The activity of acyl-CoA thioesterase may be assayed using various assays described in the state of the art. Briefly, the reaction of Ellman's reagent, which reacts with free thiol groups associated with CoASH formed upon hydrolysis of acyl-CoA may be detected by spectophotometrically monitoring absorbance at 412 nm.

According to another aspect of the present invention, there is provided a method of producing at least one acyl amino acid, comprising contacting the cell according to any aspect of the present invention with at least one fatty acid and/or amino acid.

The term "contacting", as used herein, means bringing about direct contact between the amino acid, the fatty acid and/or the cell according to any aspect of the present invention in an aqueous solution. For example, the cell, the amino acid and the fatty acid may not be in different compartments separated by a barrier such as an inorganic membrane. If the amino acid or fatty acid is soluble and may be taken up by the cell or can diffuse across biological membranes, it may simply be added to the cell according to any aspect of the present invention in an aqueous solution. In case it is insufficiently soluble, it may be solved in a suitable organic solvent prior to addition to the aqueous solution. The person skilled in the art is able to prepare aqueous solutions of amino acids or fatty acids having insufficient solubility by adding suitable organic and/or polar solvents. Such solvents may be provided in the form of an organic phase comprising liquid organic solvent. In one example, the organic solvent or phase may be considered liquid when liquid at 25 °C and standard atmospheric pressure. In another example, a fatty acid may be provided in the form of a fatty acid ester such as the respective methyl or ethyl ester. In another example, the compounds and catalysts may be contacted *in vitro,* i.e. in a more or less enriched or even purified state, or may be contacted *in situ,* i.e. they are made as part of the metabolism of the cell and subsequently react inside the cell.

The term "an aqueous solution" comprises any solution comprising water, mainly water as solvent that may be used to keep the cell according to any aspect of the present invention, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli.* It is advantageous to use as an aqueous solution a minimal medium, i.e. a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

The method according to any aspect of the present invention may be used to convert both saturated and unsaturated fatty acids, to acyl amino acids. In case the end product sought-after is to comprise a higher yield of saturated acyl residues than is present, it may be possible to complement the method according to any aspect of the present invention by hydrogenating the acyl residues of the acyl amino acids. The resultant composition would thus comprise a mixture of saturated acyl residues. The hydrogenation may be carried out according to various state of the art processes, for example those described in US5734070. Briefly, the compound to be hydrogenated may be incubated at 100 °C in the presence of hydrogen and a suitable catalyst, for example a nickel catalyst on silicon oxide as a support.

The fatty acids that are to be converted to acyl amino acids may be produced by the cell according to the present invention. In one example, the cell that produces the acyl amino acids is capable of producing the fatty acids from which the acyl amino acids are produced. In particular, the cells may be genetically modified to be able to produce fatty acids. In one example, the genetic modification may be to decrease a specific enzymatic activity and this may be done by a gene disruption or a genetic modification. The genetic modification may also increase a specific enzymatic activity. In particular, the genetic modification may increase microbial synthesis of a selected fatty acid or fatty acid derived chemical product above a rate of a control or wild type cell. This control or wild type cell may lack this genetic modification to produce a selected chemical product.

The method according to any aspect of the present invention may result in the production of fatty acyl glutamate with the amino acid being glutamic acid.

According to a further aspect of the present invention, there is provided a use of at least one cell according to any aspect of the present invention for the production of at least one acyl amino acid. In particular, the acyl amino acid may be N-acyl glutamate.

### REFERENCES

1. Antonenkov, V., D. Van Veldhoven, P., P., Waelkens, E., and Mannaerts, G.P. (1997) J. Biol. Chem. 1997, 272:26023-26031
2. Arthur Lesk (2008), Introduction to bioinformatics, 3rd edition,
3. F. M. Ausubel (1995), Current Protocols in Molecular Biology. John Wiley & Sons, Inc.
4. Barker HA, (1959) JBiol Chem. Feb;234(2):320-8.
5. Barker HA (1967) Arch. Mikrobiol. 59, 4-12
6. Blair AH, Barker HA (1966) J. Biol. Chem. 241, 400-408
7. Badenhorst CP, Drug Metab Dispos. 2012 Feb;40(2):346-52. doi: 10.1124/dmd.111.041657. Epub 2011 Nov 9.
8. Buckel W, Dorn U and Semmler R (1981) Eur. J. Biochem. 118, 315-321
9. Chen HP and Marsh ENG (1997) Biochemistry 36, 14939-14945
10. Cornish-Bowden (1995), Fundamentals of Enzyme Kinetics, Portland Press Limited, 1995
11. Duff SM, (2012) Arch. Biochem. Biophys. 528, 90-101
12. Esser D, (2013) Extremophiles, 17:205-216
13. Feng Y, (2002) Biochemistry 41. Oct 22;41 (42):12883-90
14. Fujita, Y., Matsuoka, H., and Hirooka, K. (2007) Mol. Microbiology 66(4), 829-839
15. Härtel U, (1993) Archives of Microbiology. Volume 159, Issue 2, pp 174-181
16. Hawkins AB, (2014) Appl Environ Microbiol. Apr;80(8):2536-45. doi: 10.1128/AEM.04146-13. Epub 2014 Feb 14
17. Jeffery D, (1988) Insect Biochemistry. Volume 18, Issue 4, 1988, Pages 347-349
18. Jeremy M Berg, John L Tymoczko, and Lubert Stryer, Biochemistry, 5th edition, W. H. Freeman, 2002.
19. Kalliri E, (2008) J. Bacteriol. 190, 3793-3798
20. Kang, Y., (2010), PLOS ONE 5 (10), e13557
21. Karmen (1955) J Clin Invest. Jan;34(1):131-3
22. Katoh et al., Genome Information, 16(1), 22-33, 2005.
23. Liebl W., International Journal of Systematic Bacteriology 41: 255-260 (1991)
24. Liu W, (2005) Biochemistry. Mar 1;44(8):2982-92
25. Mavrides C (1975) J. Biol. Chem. 250, 4128-4133
26. Moskowitz GJ (1969) Biochemistry. 8(7):2748-55
27. Parthasarathy A, (2011) Biochemistry. May 3;50(17):3540-50. Epub 2011 Apr 5
28. Sambrook/Fritsch/Maniatis (1989): Molecular cloning -A Laboratory Manual, Cold Spring Harbour Press, 2nd edition, Fuchs/Schlegel (2007), Allgemeine Mikrobiologie, 2008, Georg Thieme Verlag
29. Söhling B (1993) Eur J Biochem. Feb 15;212(1):121-7
30. Taylor RC, (2010) Microbiology 156, 1975-1982
31. Thompson et al., Nucleic Acids Research 22, 4637-4680, 1994,
32. Waluk, D. (2010), FASEB J. 24, 2795-2803.
33. Wang CC, (1969) J. Biol. Chem. 244, 2516-2526
34. Wiesenborn DP, (1988) Appl. Environ. Microbiol. 54, 2717-2722
35. Yamashita H, (2006) Biochim. Biophys. Acta 1761, 17-23
36. Yu K, (2011) Enzyme Microb Technol. Aug 10;49(3):272-6. doi: 10.1016/j.enzmictec.2011.06.007. Epub2011 Jun 12
37. US5734070, US20140051136

## Claims

1. A microbial cell for producing at least one acyl amino acid, wherein the cell is genetically modified to comprise:
- a first genetic mutation that enables the cell to produce the acyl amino acid; and
- a second genetic mutation that results in a decrease in activity relative to a wild type cell of at least one enzyme involved in glutamate breakdown.

2. The cell according to claim 1, wherein the acyl amino acid is N-acyl glutamate.

3. The cell according to either claim 1 or 2, wherein the first genetic mutation results in the cell having increased expression of (i) an amino acid-N-acyl-transferase (E₁) and (ii) acyl-CoA synthetase (E₂).

4. The cell according to claim 3, wherein the amino acid-N-acyl-transferase (E₁) is a glycine-N-acyl transferase (E₁ₐ) that is capable of producing N-acyl glutamate.

5. The cell according to any one of the preceding claims, wherein the enzyme involved in glutamate breakdown is selected from the group consisting of E₁₁-E₂₈.

6. The cell according to claim 5, wherein the enzyme involved in glutamate breakdown selected from the group of enzymes consisting of:
(i) E₁₁;
(ii) E₁₂, E₁₃, and E₁₄;
(iii) E₁₂, E₁₃, and E₁₅;
(iv) E₁₆;
(v) E₁₂, E₁₇, E₁₈, E₁₉, E₂₀, E₂₁, E₂₂, and E₂₃;
(vi) E₂₄, E₂₅, E₂₆, and E₂₇; and
(vii) E₂₈.

7. The cell according to any one of the preceding claims, wherein the cell further comprises a genetic mutation in at least one enzyme selected from the group consisting of:
(i) An enzyme (E₃) capable of uptake of glutamate,
(ii) An enzyme (E₄) capable of interconverting acyl-CoAs and acyl-ACPs; and
(iii) An enzyme (E₅) capable of uptake of at least one fatty acid.

8. The cell according to claim 7, wherein
- E₃ is a glutamate-translocating ABC transporter or permease;
- E₄ is acyl-CoA:ACP transacylase; and
- E₅ is AlkL and/or FadL.

9. The cell according to any of claims 3 to 8, wherein E₁ comprises SEQ ID NO: 4 or a variant thereof; and/or E₂ comprises SEQ ID NO: 1 or a variant thereof.

10. The cell according to any of the preceding claims, wherein the cell is capable of making proteinogenic amino acids and/or fatty acids.

11. The cell according to any of the preceding claims, wherein the cell has a further genetic mutation that enables the cell to have increased expression of acyl-CoA thioesterase (E₁₀).

12. A method of producing at least one acyl amino acid, comprising contacting the cell according to any one of claims 1 to 11 with at least one fatty acid and/or amino acid.

13. The method according to claim 12, wherein the amino acid is glutamic acid and the acyl amino acid is N-acyl glutamate.

14. Use of at least one cell according to any of the claims 1 to 11 for the production of at least one acyl amino acid.

15. The use according to claim 14, wherein the acyl amino acid is N-acyl glutamate.
